(19) Europäisches Patentamt
European Patent Office
Office européen des brevets

(11) Veröffentlichungsnummer: **0 439 036 A2**

(12) **EUROPÄISCHE PATENTANMELDUNG**

(21) Anmeldenummer: **91100343.2**

(22) Anmeldetag: **12.01.91**

(51) Int. Cl.$^5$: **C12Q 1/68**, G01N 33/58, C07D 475/02, C07H 21/00, G01N 33/533, C07F 15/00, C07H 19/22

(30) Priorität: **25.01.90 CH 235/90**

(43) Veröffentlichungstag der Anmeldung: **31.07.91 Patentblatt 91/31**

(84) Benannte Vertragsstaaten: **BE CH DE ES FR GB IT LI NL**

(71) Anmelder: **F. HOFFMANN-LA ROCHE AG**
**Postfach 3255**
**CH-4002 Basel(CH)**

(72) Erfinder: **Bannwarth, Wilhelm, Dr.**
**Liebenbergstrasse 8**
**W-7888 Rheinfelden-Beuggen(DE)**
Erfinder: **Müller, Francis**
**Seltisbergerstrasse 18**
**CH-4059 Basel(CH)**

(74) Vertreter: **Mezger, Wolfgang, Dr. et al**
**Grenzacherstrasse 124 Postfach 3255**
**CH-4002 Basel(CH)**

(54) **Energietransfersysteme.**

(57) Es werden Energietransfersysteme beschrieben, die u.a. für Abstandsmessungen innerhalb von oder zwischen verschiedenen Molekülen verwendet werden können.

EP 0 439 036 A2

## ENERGIETRANSFERSYSTEME

Für die Detektion sowie die Identifizierung von DNS oder RNS können Hybridisierungen mit entsprechenden komplementären Nukleotidsträngen herangezogen werden. Diese Hybridisierungen verlaufen mit sehr hoher Spezifität und weisen deswegen ein hohes Potential für die Diagnose und die Erkennung von Krankheiten auf (Methods Enzymomlogy 68, 373 [1979]).

Eine Technik zur Durchführung solcher Hybridisierungsexperimente ist die sogenannte Southern Blot Methode (J. Mol. Biol. 98, 503 [1975]), die allerdings recht umständlich ist und mit dem weiteren Nachteil behaftet, dass in der Regel radioaktive Isotope, z.B. $^{32}P$, bei dieser Technik verwendet werden. Daher sind viele Anstrengungen unternommen worden, um zum einen die Technik der Hybridisierungsverfahren zu vereinfachen und zum anderen die Radioaktivität durch geeignete nichtradioaktive Reportermoleküle zu ersetzen.

Eine Möglichkeit sowohl die Technik der Hybridisierung zu vereinfachen, als auch gleichzeitig die Radioaktivität zu ersetzen, bieten fluoreszierende Systeme, wobei ein Energietransfer von einem Donor auf einen Akzeptor stattfindet. Solche Energietransfersysteme wurden von Förster vorhergesagt (Ann. Phys. 2, 55 [1948]). Gleichzeitig wurde von Förster (supra) eine Relation zwischen der Effizienz des Energietransfers und der Entfernung zwischen Donor und Akzeptor hergestellt (sog. Förster-Gleichung). Ueberlappt die Emissionsbande des Donors die Absorptionsbande des Akzeptors, kann Energie vom Donor auf den Akzeptor übertragen werden, wobei die Effizienz dieser Energieübertragung mit der 6. Potenz der Entfernung zwischen Donor und Akzeptor abnimmt. Mit anderen Worten, je kleiner die Entfernung zwischen Donor und Akzeptor, umso intensiver ist der Energietransfer.

Inzwischen sind Energietransfermessungen zu einem hilfreichen Werkzeug für Abstandsmessungen innerhalb als auch zwischen verschiedenen Molekülen geworden (Stryer, Ann. Rev. Biochem. 47, 819 [1987]). Solche Abstandsmessungen über die Effizienz des Energietransfers zwischen Donor- und Akzeptormolekülen sind für Immunoassays als auch für DNA-Hybridisierungsassays geeignet (J. Biol. Chem. 251, 4172 [1976]; Anal. Biochem. 108, 176 [1980]; Clin. Chem. 29, 1582 [1983]; Chemiluminescent and Fluorescent Probes for DNA-Hybridisation Systems [1985]; Kingsburg, D.T. and Falkow S., Eds., 345-356, Academic Press, New York).

Die vorliegende Erfindung betrifft neue, für Abstandsmessungen innerhalb als auch zwischen verschiedenen Molekülen geeignete Energietransfersysteme bestehend aus zwei organischen Verbindungen, von denen die eine einen Chromophor vom Lumazintyp ist und die andere, mit der sie in Wechselwirkung steht, ein Rutheniumkomplex ist.

Diese Energietransfersysteme können im weiteren Sinne auch als Donor-Akzeptor-Energietransfersysteme definiert werden. Unter der Donor-Komponente sollen allgemein solche Verbindungen verstanden werden, welche in der Lage sind, Licht von einer Energiequelle aufzunehmen und anschliessend auf einen Akzeptor abzugeben. Als Akzeptor sollen allgemein solche Verbindungen verstanden werden, die befähigt sind, diese vom Donor abgegebene Energie aufzunehmen.

Gemäss vorliegender Erfindung stellen solche Akzeptoren Ru-Komplexe dar. Die Energieaufnahme erfolgt über die langwellige "metal to ligand charge transfer"-Bande (MLCT) von diesen Ru-Komplexen. Der Ausdruck "metal to ligand charge transfer"-Bande (MLCT) bezeichnet den Uebergang von einem d-Elektron des Ru (II) -Ions zu einem $\pi^*$-Elektron des Liganden-Systems des Rutheniumkomplexes. Siehe hierzu auch Crosby, J. Chem. Education 60, 791-796 [1983].

Als Chromophore vom Lumazintyp (Lu) eignen sich Lumazinderivate und ähnliche $\pi$-Systeme der allgemeinen Formel

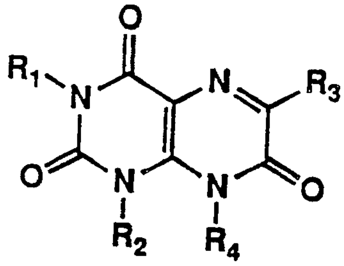

I

worin $R_1$ und $R_2$ jeweils für ein H-Atom, eine gegebenenfalls substituierte $C_{1-10}$-Alkylgruppe, 1'-Ribosyl, 1'-

(22-Desoxyribosyl) oder den Rest einer analogen Hydroxyverbindung stehen; $R_3$ für ein H-Atom oder für eine gegebenenfalls substituierte $C_{1-10}$-Alkylgruppe steht; und $R_4$ für eine gegebenenfalls substituierte $C_{1-10}$-Alkylgruppe, 1'-Ribosyl, 1' (2' -Desoxyribosyl) oder den Rest einer analogen Hydroxyverbindung steht,

oder der allgemeinen Formel

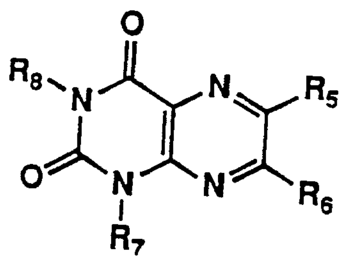

II

worin $R_5$ und $R_6$ jeweils für eine gegebenenfalls substituierte $C_{1-10}$-Alkylgruppe stehen; und $R_7$ und $R_8$ für 1'- Ribosyl, 1'(2'-Desoxyribosyl) oder den Rest einer analogen Hydroxyverbindung stehen, beispielsweise eines C-Nukleosidderivates, wie in J. Org. Chem. 54, 3927 (1989) beschrieben.

Unter analogen Hydroxyverbindungen werden Verbindungen mit einer oder mehreren Hydroxygruppen, über die sich eine kovalente Kopplung an andere Moleküle erreichen lässt, verstanden. Unter den Lumazinderivaten werden sowohl die $\alpha$-als auch die $\beta$-Anomeren verstanden. Die Zuordnung für $\alpha$- bzw. $\beta$-Anomere wurde auf Grund publizierter Daten in den einschlägigen Fachliteratur, z.B. mittels $^1$H-NMR (Chem. Ber. 106, 1401 (1973), Liebigs Ann. Chem. 1217-1234 (1977)) durchgeführt. Eigene neueste Untersuchungen mittels Röntgenstrukturanalyse zeigten, dass das ursprüngliche $\alpha$-Anomere in Wirklichkeit $\beta$-Anomeres ist und umgekehrt.

Die Rutheniumkomplexe sind Verbindungen der allgemeinen Formel

$Ru^{2+}$ $L_1$ $L_2$ $L_3$ III

wobei die Liganden $L_1$, $L_2$ und $L_3$ gleich oder verschieden sind und Ladungsübertragungseinheiten darstellen und der Ligand $L_3$ mit einer Gruppe A-X substituiert ist, wobei A eine Alkylengruppe, die auch Sulfonamid-, Thioäther-, Äther-, Carboxy- oder Carboxamidfunktionen tragen kann und X eine Aldehyd-, Carboxy-, Hydroxy-, Amino-, Thiocyanatgruppe, Halogen oder eine Phosphit- oder Phosphatgruppe oder eine modifizierte Phosphatgruppe, z.B. eine Phosphonat-oder Thiophosphatgruppe, oder eine sonstwie geeignete Funktion darstellt.

So wie hier verwendet, steht der Ausdruck "$C_{1-10}$-Alkyl" für eine gerade oder verzweigte, gegebenenfalls substituierte Alkylkette, die 1-10 C-Atome enthält, wie z.B. Methyl, Aethyl, n-Propyl, Isopropyl, n-Butyl, Isobutyl, sek. Butyl, tert. Butyl, n-Pentyl, n-Hexyl, usw. Bevorzugte Alkylgruppen sind Methylgruppen.

Unter substituierten Alkylgruppen werden solche verstanden, die die Fähigkeit zur Energieübertragung nicht negativ beeinflussen.

Beispiele für Ladungsübertragungseinheiten $L_1$, $L_2$ und $L_3$ sind Bipyridyl, Bathophenanthrolin oder Benzbathophenanthrolin, die gegebenenfalls substituiert sein können.

Die Alkylengruppe A kann gerade oder verzweigtkettig sein. Ganz besonders bevorzugt ist A eine $-(CH_2)_4-$ oder $-(CH_2)_5-$Gruppe und X eine -OH-Gruppe.

Bevorzugte erfindungsgemässe Energietransfersysteme sind DNS- oder RNS-Sequenzen, die Chromophore vom Lumazintyp in kovalenter Bindung enthalten und die entweder in modifizierter Form, vorzugsweise in aminomodifizierter Form, oder in unmodifizierter Form direkt oder über eine Spacer-Gruppe durch Reaktion mit einem Rutheniumkomplex der Formel III kovalent verknüpft sind. Bevorzugt ist eine kovalente Bindung am 5'-Ende der DNS- oder RNS-Sequenzen, am 3'-Ende oder innerhalb der DNS- oder RNS-Sequenzen, die zu diesem Zweck entsprechend modifiziert sind.

Gemäss vorliegender Erfindung können die Chromophore vom Lumazintyp am Ende der DNS- oder RNS-Sequenz oder innerhalb der DNS- oder RNS-Sequenz anstelle eines Nukleosids eingebaut werden, wobei der Einbau in beliebiger Weise erfolgen kann. Es können aber auch mehrere, vorzugsweise 2-8 aufeinanderfolgende Chromophore vom Lumazintyp am Ende der DNS- oder RNS-Sequenz, oder innerhalb der DNS- oder RNS-Sequenz, anstelle mehrerer Nukleoside eingebaut werden. Denkbar wäre es auch, die Chromophore vom Lumazintyp so zu modifizieren, dass ihre Bindung auch an entsprechend modifizierte Basen oder an das Zucker-Phosphat Rückgrat erfolgen kann.

Besonders bevorzugte erfindungsgemässe Energietransfersysteme sind:

$Ru^{2+}$ $L_1$ $L_2$ $L_3$ d(LuGTTGACAAGAATCCTCACAATACC) 3',
$Ru^{2+}$ $L_1$ $L_2$ $L_3$ d(GTLuGACAAGAATCCTCACAATACC) 3',
$Ru^{2+}$ $L_1$ $L_2$ $L_3$ d(GTTGALuAAGAATCCTCACAATACC) 3',
$Ru^{2+}$ $L_1$ $L_2$ $L_3$ d(GTTGACAALuAATCCTCACAATACC) 3', und
$Ru^{2+}$ $L_1$ $L_2$ $L_3$ d(LuLuLuLuLuGTTGACAAGAATCCTCACAATACC) 3'

bei denen der Rutheniumkomplex (Ru complex) der allgemeinen Formel III über eine sehr stabile Phosphodiesterbindung an die DNS gebunden ist und die Chromophore vom Lumazintyp 1-(2'-desoxy-α-D-ribofuranosyl)-6,7-dimethyl-lumazin (Lu) sind (siehe Figur 1).

Die Chromophore vom Lumazintyp und Rutheniumkomplexe der Formel III können aber auch in verschiedenen DNS- oder RNS-Sequenzen eingebaut als Energietransfersysteme verwendet werden. Sie können aber auch in Peptide oder Polypeptide eingebaut als Energietransfersysteme verwendet werden. Ein Einsatz in anderen Molekülarten ist ebenfalls denkbar.

Der Ausdruck "DNS- oder RNS-Sequenzen" steht für natürliche oder synthetisch hergestellte, unmodifizierte oder modifizierte DNS- oder RNS-Sequenzen.

Die Rutheniumkomplexe der allgemeinen Formel III können gemäss Europäischer Patentanmeldung, Veröffentlichungs-Nr. 178450, hergestellt werden.

Die Kopplung der Rutheniumkomplexe der allgemeinen Formel III an die DNS- oder RNS-Sequenzen, die ein oder mehrere Chromophore vom Lumazintyp enthalten, erfolgt in an sich bekannter Weise. Eine mögliche Kopplungsweise mit modifizierter DNS oder RNS besteht darin, dass man den Rutheniumkomplex der Formel III und die modifizierte DNS- bzw. RNS-Sequenz mit einem wasserlöslichen Carbodiimidderivat, z.B. mit N-Cyclohexyl-N'-(2-morpholinoäthyl)-carbodiimid-methyl-p-toluolsulfonat behandelt. Ein besonders bevorzugtes Kopplungsmittel ist 1,1,3,3-Tetramethyl-2-succinyluronium Tetrafluoroborat (1:1), nachfolgend "TSU" genannt (das vorerwähnte "TSU" kann hergestellt werden, wie es in der offengelegten japanischen Patentanmeldung Nr. 166730/86 beschrieben ist). Die Kopplung erfolgt vorzugsweise in einem Lösungsmittelgemisch, z.B. aus DMF, Dioxan oder Wasser. Ueberraschenderweise wurde gefunden, dass eine Aktivierung von Carboxylfunktionen mit TSU auch in Anwesenheit von Wasser vonstatten geht.

Die Kopplung kann aber auch direkt erfolgen, z.B. über eine Phosphodiesterbindung, die in einem Lösungsmittel, wie Acetonitril oder absolutem Pyridin ausgebildet wird. Die Rutheniumkomplexe für eine direkte Kopplung werden in eine für die Kopplung geeignete Form überführt, vorzugsweise in Phosphoramidit-, H-Phosphonat- oder aktivierte Phosphatfunktionen.

Die Phosphoramiditderivate der Rutheniumkomplexe können in situ für die Kopplung in der Oligonukleotidsynthese hergestellt werden (Bannwarth und Schmidt, Tetrahedron Letters 30, 1513 (1989)).

Die Rutheniumkomplexe in Form ihrer H-Phosphonate sind durch Umsetzung des mit einer Hydroxyalkylgruppe derivatisierten Rutheniumkomplexes mit Trisimidazoylphosphin (Fröhler et al., Nucleic Acid Research 14, 5399 (1986)) oder mit Salizylchlorophosphin als Reagenz (Marugg et al., Tetrahedron Letters 27, 2271 (1986)) und nachfolgender Hydrolyse erhältlich. Die Herstellung kann gemäss Figur 6 erfolgen. Die so erhaltenen H-Phosphonate lassen sich in bekannter Weise in der Synthese von Oligonukleotiden verwenden (Garreg et al., Chemica Scripta 25, 280 (1985)).

Die Herstellung der DNS- oder RNS-Sequenzen, die ein oder mehrere Chromophore vom Lumazintyp anstelle einer oder mehrerer Nukleoside eingebaut haben oder zusätzlich an einem Ende der DNS- oder RNS-Sequenz enthalten, erfolgt in an sich bekannter Weise. Die Chromophore vom Lumazintyp werden in eine für die Kopplung geeignete Form überführt, vorzugsweise in Phosphoramidit-, H-Phosphonat- oder aktivierte Phosphatfunktionen. Die Kopplung erfolgt vorzugsweise an das wachsende DNS- oder RNS-Fragment während der Synthese. Die Synthese kann sowohl in flüssiger Phase als auch an fester Phase durchgeführt werden, wie sie beispielsweise in Science 230, 281 (1985), Chimia 41, 302 oder in "Oligonucleotide Synthesis: A practical Approach", IRL Press, Oxford, UK, M.J. Gait, Ed. (1984) beschrieben ist.

Besonders bevorzugte DNS-Sequenzen, in welche ein oder mehrere Chromophore vom Lumazintyp anstelle einer oder mehrerer Nukleoside oder zusätzlich am 5'-Ende eingebaut sind, sind:

d(GTLuGACAAGAATCCTCACAATACC) 3',
d(GTTGALuAAGAATCCTCACAATACC) 3',
d(GTTGACAALuAATCCTCACAATACC) 3',
d(LuGTTGACAAGAATCCTCACAATACC) 3', und
d(LuLuLuLuLuGTTGACAAGAATCCTCACAATACC) 3'

worin Lu für 1-(2'-desoxy-α-D-ribofuranosyl)-6,7-dimethyl-lumazin steht.

Diese DNS- oder RNS-Sequenzen bilden auch einen Gegenstand der vorliegenden Erfindung.

Ebenfalls einen Gegenstand der vorliegenden Erfindung bilden die Lumazinderivate und ähnliche π-Systeme der allgemeinen Formel

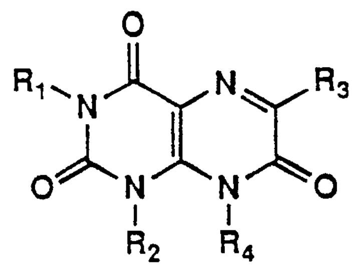

I

worin $R_1$ und $R_2$ jeweils für ein H-Atom, eine gegebenenfalls substituierte $C_{1-10}$-Alkylgruppe, 1'-Ribosyl, 1'-(2'-Desoxyribosyl) oder den Rest einer analogen Hydroxyverbindung stehen; $R_3$ für ein H-Atom oder für eine gegebenenfalls substituierte $C_{1-10}$-Alkylgruppe steht; und $R_4$ für eine gegebenenfalls substituierte $C_{1-10}$-Alkylgruppe, 1'-Ribosyl, 1'(2'-Desoxyribosyl) oder den Rest einer analogen Hydroxyverbindung steht, oder der allgemeinen Formel

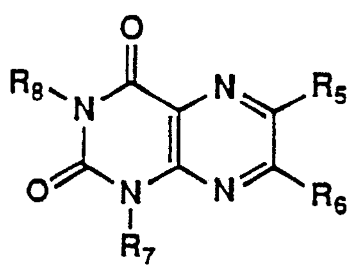

II

worin $R_5$ und $R_6$ jeweils für eine gegebenenfalls substituierte $C_{1-10}$-Alkylgruppe stehen; und $R_7$ und $R_8$ für 1'-Ribosyl, 1'(2'-Desoxyribosyl) oder den Rest einer analogen Hydroxyverbindung stehen, beispielsweise eines C-Nukleosidderivates, wie in J. Org. Chem. 3927, 16 (1989) beschrieben.

In einer bevorzugten Ausführungsform hat das Lumazinderivat die folgende Formel:

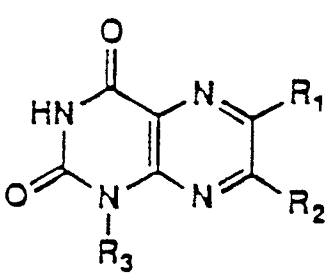

II-1

worin $R_1$ und $R_2$ für eine gegebenenfalls substituierte $C_{1-10}$-Alkylgruppe, vorzugsweise eine Methylgruppe, steht; und $R_3$ für 1'-Ribosyl oder 1'(2'-Desoxyribosyl) steht.

Ein besonders bevorzugtes Lumazinderivat ist 1-(2'-desoxy-α-D-ribofuranosyl)-6,7-dimethyl-lumazin (siehe Verbindung 1 in Figur 2).

Die vorstehend genannten Lumazinderivate können wie von Ritzmann und Pfleiderer in Chem. Ber. 106, 1401 (1973) beschrieben, hergestellt werden oder in analoger Weise hierzu.

Ein weiterer Gegenstand der vorliegenden Erfindung bilden Phosphoramidite und H-Phosphonate der vorstehend genannten Lumazinderivate, die für die Festphasen-oder Lösungssynthesen von Oligo- oder

Polynukleotiden geeignet sind.

Ein besonders bevorzugtes Phosphoramidit der vorliegenden Erfindung ist 1-(5'-O-4,4'-Dimethoxytrityl-2'-desoxy-α-D-ribofuranosyl-3'-O-((2-cyanoethyl)-N,N-diisopropyl-phosphoramidit)-6,7-dimethyl-lumazin (siehe Verbindung 3 in Figur 2).

Die vorstehend genannten Phosphoramidite oder H-Phosphonate der Lumazinderivate können durch Umsetzung von am 5'-Ende geschützten, vorstehend genannten Lumazinderivaten nach bekannten Methoden hergestellt werden. Vorzugsweise wird das 5'-Ende des Lumazinderivats zuerst durch eine 4,4'-Dimethoxytritylgruppe geschützt und die resultierende Verbindung anschliessend mit 2-Cyanoethoxy-bis-diisopropylaminophosphin in Gegenwart von Diisopropylammoniumtetrazolid in das entsprechende Phosphoramidit des Lumazinderivates umgesetzt. Das hier bereits genannte, besonders bevorzugte Phosphoramidit 1-(5'-O-4,4'-Dimethoxytrityl-2'-desoxy-α-D-ribofuranosyl-3'-O-((2-cyanoethyl)-N,N-diisopropyl-phosphoramidit)-6,7-dimethyl-lumazin 3 kann gemäss Figur 2 hergestellt werden.

Ein weiterer Gegenstand der vorliegenden Erfindung sind Lumazinderivate, die den Einbau von Lumazinen am 3'-Ende von Oligonukleotiden in der Festphasensynthese ermöglichen.

Ein besonders bevorzugtes Lumazinderivat der vorliegenden Erfindung ist 1-(5'-0-4,4'-Dimethoxytrityl-2'-desoxy-α-D-ribofuranosyl-3'-0-succinyl)-6,7-dimethyl-lumazin (siehe Verbindung 16 in Figur 5).

Die vorstehend genannte Verbindung kann durch Umsetzung des am 5'-Ende mit einer 4,4'-Dimethoxytritylgruppe geschützten Lumazinderivats 2 nach bekannten Methoden hergestellt werden. Vorzugsweise wird das geschützte Lumazinderivat mit Bernsteinsäureanhydrid unter Aktivierung umgesetzt und das Produkt als Salz der Säure isoliert. Die Herstellung des 1-(5'-0-4,4'-Dimethoxytrityl-2'-desoxy-α-D-ribofuranosyl-3'-0-succinyl)-6,7-dimethyl-lumazin kann gemäss Figur 5 erfolgen.

Ein weiterer Gegenstand der vorliegenden Erfindung ist mit Lumazinderivaten modifiziertes Controlled-Pore-Glas (CPG) (siehe Verbindung 17 in Figur 5), welches üblicherweise als Trägermaterial in der Festphasensynthese verwendet wird. Andere für die Modifizierung verwendbare Trägermaterialien, wie z.B. Kieselgel können ebenfalls benutzt werden (F. Chow; T. Kempe; G. Palm; Nucleic Acids Res. 9, 2807 [1981]).

Durch die Verknüpfung des CPG-Materials mit Nukleosiden und deren Derivate können die verschiedensten Reste am 3'-Ende eines Oligonukleotides eingebaut werden. In der bevorzugten Ausführungsform der vorliegenden Erfindung wird das Trägermaterial mit einem Lumazinderivat verknüpft, welches für den weiteren Aufbau eines Oligonukleotids geeignet ist.

Die Herstellung des funktionell modifizierten CPG erfolgt in an sich bekannter Weise durch Kupplung des 1-(5'-0-4,4'-Dimethoxytrityl-2'-desoxy-α-D-ribofuranosyl-3'-0-succinyl) 6,7-dimethyl-lumazin 16 mit für die Verknüpfung geeignetem CPG-Material. In der bevorzugten Ausführungsform erfolgt die Herstellung des funktionalisierten Trägermaterials gemäss Figur 5 durch Aktivierung von 16 mit Mesitylen-2-sulfochlorid (MsCl) und 1-Methyl-imidazol (MeIm).

Mit diesem so erhaltenen Träger lassen sich Lumazinderivate am 3'-Ende von Oligo- oder Polynukleotiden einführen. Dies hat den Vorteil, dass zum Beispiel Energietransfersysteme zwischen Oligonukleotiden möglich sind, die mit Donor oder Akzeptor an verschiedenen Enden der Oligonukleotide ausgerüstet sind.

Besonders bevorzugte erfindungsgemässe Energietransfersysteme mit diesen Eigenschaften sind:

5'-d(TGGGATAGGTGGATTAT-LuLuLuLu) 3'
5'-d(CTACTGGGATAGGTGGA-LuLuLuLu) 3'
5'-d(TCAACGTATGTTCACCG-LuLuLuLu) 3'

Ueberraschenderweise hat sich gezeigt, dass die Chromophore vom Lumazintyp gemäss vorliegender Erfindung geeignet sind, Licht von einem Stickstoff-Laser auf Rutheniumkomplexe der Formel III zu übertragen.

Die Kombinationen Chromophor vom Lumazintyp/Rutheniumkomplex der Formel III stellen damit Energietransfersysteme dar (mit dem Chromophor vom Lumazintyp als Donor und dem Rutheniumkomplex der Formel III als Akzeptor), die für Abstandsmessungen innerhalb eines oder zwischen verschiedenen Molekülen ausserordentlich geeignet sind, da, wie bereits einleitend ausgeführt, auf Grund der Förstergleichung eine strenge Korrelation zwischen Energietransfer und dem Abstand zwischen Donor und Akzeptor besteht.

Solche Abstandsmessungen können zur Bestimmung von Molekülassoziationen zwischen verschiedenen Molekülen, beispielsweise zwischen DNS- oder RNS-Sequenzen und Proteinen/Peptiden herangezogen werden, wenn eine Molekülart mit dem Donor und die andere mit dem Akzeptor ausgestattet ist. Dies kann zum Nachweis von Interaktionen dieser Moleküle und zum Nachweis der Anwesenheit oder Abwesenheit

von Molekülen herangezogen werden. Diese Nachweise eignen sich im besonderen für diagnostische Tests, wie z.B. Immunoassays, Rezeptorscreeningassays, DNS-Sonden-Assays.

Eine Kopplung der Ru-Komplexe der Formel III an Proteine/Peptide wurde in der Europäischen Patentanmeldung, Veröffentlichungs-Nr. 178 450, beschrieben. Die Modifizierung von Lumazin-2'-desoxyribosiden am 5'-Ende mit einer Aminofunktion kann analog derjenigen von Thymidin (Helv. Chim. Acta 71, 1517 [1988]) erfolgen. Damit ist die Kopplung von Chromophoren vom Lumazintyp an Proteine/Peptide über Carboxamidbindungen zu bewerkstelligen. Dadurch lässt sich das erfindungsgemässe Energietransfersystem zwischen einem Chromophor vom Lumazintyp und einem Ru-Komplex der Formel III in solchen Tests anwenden, bei welchen Abstände zwischen Proteinen/Peptiden eine Rolle spielen, wie etwa bei Immunoassays.

Auch Rezeptorscreeningassays können auf dieser Basis aufgebaut werden.

Durch den Einbau des Energietransfersystems im gleichen Molekül lassen sich Abstandsmessungen innerhalb eines Moleküls durchführen. Durch den Einbau der Komponenten des Energietransfersystems in verschiedene Moleküle lassen sich aber auch Abstandsmessungen zwischen verschiedenen Molekülen durchführen. Solche Systeme stellen die bevorzugten erfindungsgemässen Energietransfersysteme dar.

Darüber hinaus sind die besagten Donoren, insbesondere die erfindungsgemässen Lumazinderivate, geeignet, in der für die Anregung der Rutheniumkomplexe verwendeten Kombination von Stickstoff- und Farbstofflaser letzteren zu ersetzen.

Die Methodik der zeitaufgelösten Fluoreszenz-Technik ist beispielsweise in der DTOS 2628158 oder der früher erwähnten Europäischen Patentanmeldung Nr. 85.1113777.9 (Veröffentlichungs-Nr. 178450) beschrieben.

Die nachfolgenden Beispiele und Figuren illustrieren die vorliegende Erfindung ohne sie zu beschränken.

Figur 1 zeigt die Struktur des 5'-Endes der Verbindung 11 (Beispiel 4).

Figur 2 zeigt schematisch die Herstellung von 1-(5'-O-4,4'-Dimethoxytrityl-2'-desoxy-α-D-ribofuranosyl-3'-O-(2-cyanoethyl)-N,N-diisopropyl-phosphoramidit)-6,7-dimethyl-lumazin (3).

Figur 3 zeigt schematisch die Synthese von verschiedenen DNS-Sequenzen, die ein oder mehrere Chromophore vom Lumazintyp anstelle einer oder mehrerer Nukleoside eingebaut haben oder zusätzlich am 5'-Ende der DNS-Sequenz enthalten.

Figur 4 zeigt die gelelektrophoretische Analyse der Verbindungen 4-9 (Beispiel 3) und 10-15 (Beispiel 4).

Figur 5 zeigt schematisch die Synthese des 1-(5'-0-4,4'-Dimethoxytrityl-2'-desoxy-α-D-ribofuranosyl-3'-0-succinyl)-6,7-dimethyl-lumazin und des mit Lumazin modifizierten Trägers.

Figur 6 zeigt die Synthese des Ru-Komplex H-Phosphonats.

Figur 7 zeigt die Sequenzen der Oligonukleotide 20-25 und ihre Verwendung in Energietransfersystemen.

Figur 8 zeigt die Sequenzen der Oligonukleotide 20, 21, 23, 24, 25 und ihre Verwendung in Energietransfersystemen.

## Beispiele

Alle Lösungsmittel waren von höchster Reinheit. Das Phosphoramidit des Ruthenium (bathophenanthrolin)-Komplexes wurde in situ, wie von W. Bannwarth und D. Schmidt beschrieben (Tetrahedron Lett. 30, 1513, 1989), hergestellt. DNS-Synthesen wurden an festen Trägern (Adams et al., J. Am. Chem. Soc. 105, 661 [1983]) mittels Phosphoramidit-Chemie nach publizierten Methoden, z.B. nach Sinha et al., Nucleic Acids Res. 12, 4539 (1984) oder Bannwarth, Chimia 41, 302 (1987), durchgeführt. Zeitaufgelöste Fluoreszenzmessungen wurden in einem Volumen von 100 μl mittels eines publizierten Apparates [Europäische Patentanmeldung, (Veröffentlichungs-Nr. 178450)] durchgeführt. Kurzsäulenchromatographie (CC), wie von Hunt und Rigby beschrieben (Chem. Ind., London, 1868 [1967]), wurde mit Silica Gel 60 (0.063-0.040 mm, Merck) durchgeführt. 1-(2'-desoxy-α-D-ribofuranosyl)-6,7-dimethyl-lumazin (1) wurde, wie von Ritzmann und Pfleiderer in Chem. Ber. 106, 1401 (1973) beschrieben, hergestellt.

### Beispiel 1

1-(5'-O-4,4'-Dimethoxytrityl-2'-desoxy-α-D-ribofuranosyl)-6,7-dimethyl-lumazin (2)

0.15 mmol (45 mg) der Verbindung 1 wurden zweimal in abs. Pyridin aufgenommen und evaporiert. Dann wurde erneut in abs. Pyridin (5 ml) gelöst und mit 0.25 mmol (85 mg) 4,4'-Dimethoxytritylchlorid versetzt und bei Raumtemperatur (RT) gerührt. Nach 1 h wurde 1 ml Methanol zugegeben und nach weiteren 15 Min. in gesättigte $NaHCO_3$-Lösung gegossen und dreimal mit je 30 ml Methylenchlorid ($CH_2Cl_2$) extrahiert. Die vereinigten organischen Phasen wurden über $Na_2SO_4$ getrocknet und nach Filtration vom Trockenmittel eingeengt. Der Rückstand wurde über 10 g Kieselgel mittels Kurzsäulenchromatographie mit 100 ml $CH_2Cl_2/Et_3N$ (99/1) und 100 ml $CH_2Cl_2/MeOH/Et_3N$ (97/2/1) aufgetrennt. Die reinen Produktfraktionen wurden gesammelt und eingeengt. Der Rückstand wurde nach Lösen in 5 ml Chloroform durch Eintropfen in 150 ml n-Pentan gefällt. Das Präzipitat wurde gesammelt und getrocknet und lieferte 65 mg (48.3%) reines Produkt.

Beispiel 2

1-(5'-O-4,4'-Dimethoxytrityl-2'-desoxy-α-D-ribofuranosyl-3'-O-((2-cyanoethyl)-N,N-diisopropyl-phosphoramidit)-6,7-dimethyl-lumazin (3)

0.3 mmol (183 mg) der Verbindung 2 (Beispiel 1) wurden in 15 ml MeCN (abs.) aufgenommen und evaporiert. Der Rückstand wurde dann erneut in 15 ml MeCN aufgenommen, mit 0.6 mmol (180 mg) 2-Cyanoethoxy-bis-diisopropylaminophosphin und 0.3 mmol (51 mg) Diisopropylammonium tetrazolid versetzt und für 2 h gerührt. Danach wurde die Mischung in 100 ml gesättigte $NaHCO_3$-Lösung gegossen und dreimal mit je 30 ml $CH_2Cl_2$ extrahiert. Die vereinigten organischen Phasen wurden über $Na_2SO_4$ getrocknet und nach Filtration vom Trockenmittel eingeengt. Der Rückstand wurde über 10 g Kieselgel mittels Kurzsäulenchromatographie mit 100 ml $CH_2Cl_2/Et_3N$ (99/1) und 100 ml $CH_2Cl_2/Et_3N$ (98/2) aufgetrennt. Die reinen Produktfraktionen lieferten 170 mg (70%) reines Produkt.

Beispiel 3

Synthese von d(GTTGACAAGAATCCTCACAATACC) $_{3'}$, 4,
d(GTLuGACAAGAATCCTCACAATACC) $_{3'}$, 7,
d(GTTGALuAAGAATCCTCACAATACC) $_{3'}$, 6,
d(GTTGACAALuAATCCTCACAATACC) $_{3'}$, 5,
d(LuGTTGACAAGAATCCTCACAATACC) $_{3'}$, 8 und
d(LuLuLuLuLuGTTGACAAGAATCCTCACAATACC) $_{3'}$, 9.

Die Synthese wurde mit 180 mg C (4.87 μmol) funktionalisiertem Controlled Pore Glas als festem Trägermaterial begonnen (Fig. 3). Die Kettenverlängerungen wurden mit jeweils 40 mg des entsprechenden β-Cyanoethylphosphoramidits bis zum Einbau des Lumazindesoxyribosids 3 (Beispiel 2) durchgeführt. Immer wenn ein Lumazindesoxyribosid Phosphoramidit 3 eingebaut wurde, wurden 30 mg festes Trägermaterial mit der entsprechenden Sequenz abgetrennt und die Synthese gemäss Figur 3 fortgeführt. Für jede Kettenverlängerung mit 3 wurden 20 mg dieses Amidits verwendet. Von jeder geschützten und noch an das feste Trägermaterial gekoppelten Sequenz wurden 8 mg abgetrennt und nach Schutzgruppenabspaltung mit Ammoniak auf einem 20% Polyacrylamidgel unter denaturierenden Bedingungen aufgetrennt (Figur 4).

Beispiel 4

Synthese von

Ru Komplex-d(GTTGACAAGAATCCTCACAATACC) $_3$, 10,
Ru Komplex-d(GTLuGACAAGAATCCTCACAATACC) $_3$, 12,
Ru Komplex-d(GTTGALuAAGAATCCTCACAATACC) $_3$, 13,
Ru Komplex-d(GTTGACAALuAATCCTCACAATACC) $_3$, 14,
Ru Komplex-d(LuGTTGACAAGAATCCTCACAATACC) $_3$, 11 und
Ru Komplex d(LuLuLuLuLuGTTGACAAGAATCCTCACAATACC) $_3$, 15.

Nach Abspaltung der Dimethoxytrityl-Schutzgruppe wurde der Bathophenanthrolin-Ruthenium II-Komplex in Form seines in situ hergestellten Phosphoramidits mit den Verbindungen 4-9 (Beispiel 3) gekoppelt (Bannwarth and Schmidt, Tetrahedron Letters 30, 1513 (1989)). Durch Behandlung mit conc. Ammoniak wurden dann die Verbindungen 10-15 in ungereinigter Form erhalten. Figur 4 zeigt die Analyse dieser Verbindungen mittels 20% Polyacrylamid-Gelelektrophorese.

Die Verbindungen 10-15 in reiner Form wurden entweder mittels Umkehrphasen-HPLC oder mittels präparativer Gelelektrophorese und nachfolgender Elektroelution erhalten.

Beispiel 5

Fluoreszenzmessungen mit den Verbindungen 11-15 zur Bestimmung der Effizienz des Energietransfers

Die Fluoreszenzintensitäten der Verbindungen 11-15 wurden mittels zeitaufgelöster Fluoreszenz-Technik gemessen. Die Verbindungen 11-15 wurden mittels Lichtpulsen eines Stickstofflasers (0.7 ns bei 337 nm) angeregt und das Fluoreszenzlicht wurde mittels eines Photoverstärkers gemessen.

Die gemessene Intensität der Fluoreszenz $I_F$ bei 618 nm (Emissionswellenlänge des Rutheniumkomplexes) kann durch die Summe von 3 Komponenten beschrieben werden:

$$I_F = I_{F1} + I_{F2} + I_{F3}.$$

$I_{F1}$ ist die Fluoreszenzintensität des Lumazin bei 618 nm. $I_{F2}$ stellt die durch direkte Anregung verursachte Emission des Ru Komplexes dar und $I_{F3}$ bezeichnet den Fluoreszenzintensitäts-Beitrag des Energietransfers. Da $I_{F1}$ praktisch null ist, lässt sich die gemessene Fluoreszenzintensität so schreiben:

$$I_F \sim I_{F2} + I_{F3}.$$

In der folgenden Tabelle sind die Effizienzen (E) des Energietransfers der Verbindungen 10-15 aufgeführt. Diese wurden nach der Formel

$$E = \frac{I_F - I_{F2}}{I_{F2}} \cdot 100 \ [\%]$$

berechnet.

| Verbindung | E (%) |
|---|---|
| 11 | 15.0 |
| 12 | 12.6 |
| 13 | 5.4 |
| 14 | 4.1 |
| 15 | 77.0 |

Die Tabelle offenbart eine Korrelation zwischen der Effizienz des Energietransfers und dem Abstand zwischen Donor (Chromophor vom Lumazintyp) und Akzeptor (Rutheniumkomplex). Ausserdem lässt sich durch den Einbau mehrerer Donoren die Effizienz des Transfers steigern, wie mit Verbindung 15 gezeigt.

Beispiel 6

Synthese von 5' d(TGGGATAGGTGGATTAT-LuLuLuLu) 3' (20)

A. Synthese von 1-(5'-0-4,4'-Dimethoxytrityl-2'-desoxy-α-D-ribofuranosyl-3'-0-succinyl)-6,7-dimethyl-lumazin (16).

0,29 mmol (175 mg) der Verbindung 2 (Beispiel 1) wurden mehrmals in trockenem Pyridin aufgenommen und evaporiert. Der Rückstand wurde in 5 ml trockenem $CH_2Cl_2$ aufgenommen und 0,91 mmol (91 mg) Bernsteinsäureanhydrid, 0,33 mmol (41 mg) DMAP sowie 0,91 mmol (126 μl) $Et_3N$ zugegeben und die Mischung für fünf Stunden bei Raumtemperatur gerührt. Die Mischung wurde in 10 ml 1%ige Essigsäure gegossen und dreimal mit je 50 ml $CH_2Cl_2$ extrahiert. Die vereinigten organischen Phasen wurden getrocknet und eingeengt. Der Rückstand wurde über 20 g Kieselgel durch Säulenchromatographie mit $CH_2Cl_2/Et_3N$ (99/1) und steigendem Ethanolgradient (1,3, 5%) gereinigt. Reine Fraktionen wurden gesammelt und eingeengt. Das Produkt (DC; Rf: 0,32; $CH_2Cl_2$/MeOH 9/1) wurde in 5 ml $CH_2CH_2$/1% $NEt_3$ gelöst und in 350 ml n-Pentan gefällt. Der Niederschlag wurde gesammelt und getrocknet. Ausbeute: 165 mg (79%) von 16 als Triethylammoniumsalz.

B. Funktionalisierung des CPG-Trägers mit 1-(5'-0-4,4'-Dimethoxytrityl-2'-desoxy-α-D-ribofuranosyl-3'-0-succinyl)-6,7-dimethyl-lumazin (16) zu Verbindung 17.

2,5 g des CPG-Trägers und 0,18 mmol (130 mg) von Verbindung 16 wurden zusammen in 10 ml trockenem Pyridin mehrmals evaporiert. Der Rückstand wurde in 10 ml trockenem Pyridin aufgenommen

und 4,6 mmol (1,0 g) Mesitylen-2-Sulfochlorid sowie 4 mmol (0,4 ml) 1-Methyl-Imidazol dazugegeben. Während der Reaktionszeit (18 Stunden) wurde gelegentlich umgeschüttelt. Das Gemisch wurde filtriert und mit Pyridin und Äther gewaschen. Nichtabreagierte Aminogruppen wurden durch Zugabe von 15 ml einer Lösung aus 1 g DMAP, 2 ml $Ac_2O$ und 2 ml Lutidin blockiert. Nach 30 Minuten wurde die Lösung abfiltriert und der funktionalisierte Träger mit Pyridin und Äther gewaschen und getrocknet. Durch UV-Messung der abgespaltenen DMTr-Gruppe (499 nm) wurde eine Beladung des Trägers (17) von 27 µmol/g ermittelt.

C. Synthese des 3'-lumazinderivatisierten Oligonukleotids 5' d(TGGGATAGGTGGATTATLuLuLuLu)3' (20).

Die Synthese des Oligonukleotids erfolgte mit dem in Teil B. hergestellten Träger (17) unter Verwendung des Lumazinphosphoramidites 3 aus Beispiel 2. Dazu wurden 1,08 µmol (40 mg) des Trägers mit 24 µmol (20 mg) der Verbindung 3 und 240 µmol (17 mg) Tetrazol in 0,5 ml trockenem MeCN in einem Standardreaktionszyklus gekuppelt. Nach dem Einbau der Lumazinchromophoren am 3'-Ende wurde die Synthese mit normalen Phosphoramiditen fortgesetzt. Nach Entschützung mit $NH_3$ erfolgte die Isolierung des Oligonukleotids (20) durch Elektroelution nach präparativer Gelelektrophorese.

Beispiel 7

## Synthese von Ru Komplex-d(ATAATCCACCTATCCCAGTAGGAGAAAT) 3'(21)

A. Synthese des H-Phosphonats des Bathophenanthrolin Ru (III) Komplexes (18).

Verfahren A:

In eine Lösung von 2,7 mmol (180 mg) Imidazol und 2,8 mmol (380 µl) $Et_3N$ in 10 ml trockenem MeCN wurde unter Argon innerhalb von 5 Minuten mit einer Spritze 0,8 mmol (70 µl) $PCl_3$ zugegeben und die Mischung für 30 Minuten bei Raumtemperatur (RT) gerührt. Separat dazu wurden 0,1 mmol (126 mg) des Ru-Komplexes mit trockenem MeCN evaporiert und nach Aufnahme in 10 ml MeCN zu der Mischung mit dem Trisimidazoylphosphin gegeben. Nach 2h Rühren bei RT wurde die Mischung in 100 ml 0,1 M TEAA pH 7,0 gegossen und mit $CH_2Cl_2$ (3x50 ml) extrahiert. Die vereinigten organischen Phasen wurden über $Na_2SO_4$ getrocknet. Nach Einengen wurde der Rückstand mit Äther digeriert und anschliessend gewaschen. Nach Trocknen wurden 120 mg (86%) des H-Phosphonats 18 erhalten.

Verfahren B:

0,1 mmol (126 mg) des Ru-Komplexes wurden mit MeCN evaporiert, in 5 ml trockenem MeCN und 1 ml trockenem Pyridin gelöst und eine Lösung von 0,5 mmol (101 mg) Salizylchlorophosphin in 2 ml trockenem MeCN dazugegeben. Diese Reaktionsmischung wurde bei RT für 1,5 h gerührt, in 50 ml 0,1 M TEAA gegossen und, wie in Verfahren A beschrieben, aufgearbeitet. Ausbeute: 120 mg (86%) des H-Phosphonats 18.

B. Kopplung von Ru Komplex H-Phosphonaten an Oligonukleotide.

Das Oligonukleotid mit der Sequenz d(ATAATCCACCTATCC-CAGTAGGAGAAAT) 3' wurde in bekannter Weise hergestellt und am 5'-Ende entschützt am Träger belassen. 26 µmol (36 mg) Verbindung (18) wurden mit trockenem MeCN evaporiert und in 1 ml trockenem Pyridin aufgelöst. 0,5 ml dieser Lösung wurden gleichzeitig mit 7,3 µl Pivaloylchlorid, gelöst in 0,5 ml trockenem MeCN, zu 0,4 µmol des trägergebundenen Oligonukleotids d(ATAATCCACCTATCCCAGTAGGAGAAAT) 3' gegeben. Nach einer Kupplungszeit von 4 Minuten wurde der Träger gewaschen und nochmals mit der gleichen Menge an Reagenzien umgesetzt. Danach erfolgte die Oxidation mit 1 ml 0,2 M $J_2$/THF und 1 ml $Et_3N/H_2O$/THF (1:8:1, v/v) und Waschschritte mit MeCN und Äther. Zum Entschützen wurden 10 mg des Trägers mit 700 µl konz. Ammoniak für zwei Stunden bei 67°C behandelt. Durch Polyacrylamid-Gelelektrophorese konnte die vollständige Umwandlung des Startoligonukleotids in das mit dem Ru Komplex markierte Oligonukleotid (21) gezeigt werden.

Beispiel 8

## Fluoreszenzmessungen mit den Verbindungen 20-25 zur Bestimmung der Effizienz des Energietransfers

Um die Energieübertragung zwischen Lumazin und Ru-Komplex, die jeweils mit verschiedenen Molekülen verknüpft sind, zu bestimmen, wurden die Oligonukleotide (20) und (23) sowie das Ru-Komplex markierte Oligonukleotid (21) an ein synthetisches Templat (22) hybridisiert und die Energieübertragung bestimmt.

Die Oligonukleotide (24) und (25) wurden als negative Kontrollen (keine Energieübertragung) eingesetzt (siehe Fig. 7). Zur Charakterisierung des Energietransfers sind in der folgenden Tabelle die Verhältnisse der gemessenen Fluoreszenzintensitäten $I_F$ und $I_{F2}$ der getesteten Oligonukleotide aufgeführt. Dabei werden für $I_F$ und $I_{F2}$ die Definitionen von Beispiel 5 herbeigezogen.

| Hybrid | $I_F/I_{F2}$ |
|---|---|
| 22/21/25 | 1,0 |
| 22/21/24 | 1,1 |
| 22/21/23 | 1,9 |
| 22/21/20 | 2,2 |

In den Fällen, in denen die Oligonukleotide 24 und 25 verwendet wurden (negative Kontrollen), war keine Energieübertragung zu beobachten ($I_F/I_{F2} \sim 1$). Bei passender Hybridisierung (Hybrid 22/21/20 und 22/21/23) war ein Energietransfer möglich, der durch die Verdoppelung der Signalintensität für das Fluoreszenzlicht aus dem Energietransfer angezeigt wurde ($I_F/I_{F2} \sim 2$).

Somit war durch die Messung des Energietransfers eine klare Aussage möglich, ob eine Hybridisierung der beiden Donor/ Akzeptor Oligonukleotide an ein gesuchtes Target, in diesem Fall eine DNA-Sequenz, stattgefunden hatte.

Die gleichen Sequenzen 21-25 wurden auch dazu verwendet, den Energietransfer zwischen zwei getrennten Molekülen zu bestimmen, ohne dass diese, wie oben beschrieben, über ein drittes Molekül fixiert werden mussten (Fig. 8). Es wurde dabei nur die Spezifität der Erkennung zwischen den beiden Verbindungen des Energietransfersystems ausgenützt. Im vorliegenden Fall hybridisierten die Oligonukleotide 21/20 und 21/23 aufgrund ihrer Basenzusammensetzung miteinander und bildeten mit ihren Chromophoren ein Energietransfersystem. Demgegenüber konnte das mit einem Chromophor markierten Oligonukleotid 24 nicht mit 21 hybridisieren. Das Oligonukleotid 25 hybridisierte zwar mit 21, es trug aber kein für ein Energietransfersystem benötigtes Chromophor. Wie die unterstehende Tabelle zeigt, ergaben sich bei den Fluoreszenzmessungen nur bei den Paaren 21/20 und 21/23 die erwarteten Energietransfers, während die anderen beiden Paare 21/24 und 21/25 keine Transfers zeigten (Kontrollen).

| Hybrid | $I_F/I_{F2}$ |
|---|---|
| 21/20 | 1,9 |
| 21/23 | 1,8 |
| 21/24 | 1,0 |
| 21/25 | 1,0 |

## Patentansprüche

1. Energietransfersysteme bestehend aus zwei organischen Verbindungen, von denen die eine ein Chromophor vom Lumazintyp ist und die andere, mit der sie in Wechselwirkung steht, ein Ru-Komplex ist.

2. Energietransfersysteme nach Anspruch 1, dadurch gekennzeichnet, dass das Chromophor vom Lumazintyp ein Lumazinderivat der allgemeinen Formel

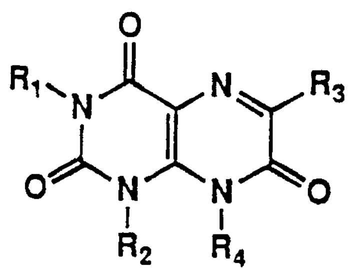

I

ist, worin $R_1$ und $R_2$ jeweils für ein H-Atom, eine gegebenenfalls substituierte $C_{1-10}$-Alkylgruppe, 1'-Ribosyl, 1'(2'-Desoxyribosyl) oder den Rest einer analogen Hydroxyverbindung stehen; $R_3$ für ein H-Atom oder für eine gegebenenfalls substituierte $C_{1-10}$-Alkylgruppe steht; und $R_4$ für eine gegebenenfalls substituierte $C_{1-10}$-Alkylgruppe, 1'-Ribosyl, 1'(2'-Desoxyribosyl) oder den Rest einer analogen Hydroxyverbindung steht;
oder der allgemeinen Formel

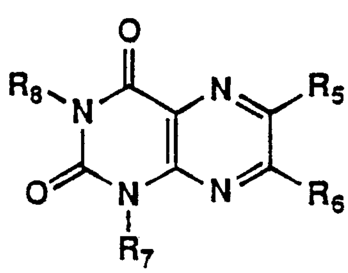

II

ist, worin $R_5$ und $R_6$ jeweils für eine gegebenenfalls substituierte $C_{1-10}$-Alkylgruppe stehen; und $R_7$ und $R_8$ für 1'-Ribosyl, 1'(2'-Desoxyribosyl) oder den Rest einer analogen Hydroxyverbindung stehen.

3. Energietransfersysteme nach Anspruch 1 oder 2, dadurch gekennzeichnet, dass der Rutheniumkomplex eine Verbindung der allgemeinen Formel

$Ru^{2+}$ $L_1$ $L_2$ $L_3$ III

ist, wobei die Liganden $L_1$, $L_2$ und $L_3$ gleich oder verschieden sind und Ladungsübertragungseinheiten darstellen, und der Ligand $L_3$ mit einer Gruppe A-X substituiert ist, wobei A eine Alkylengruppe, die auch Sulfonamid-, Thioäther-, Äther-, Carboxy- oder Carboxamidfunktionen tragen kann und X eine Aldehyd-, Carboxy, Hydroxy, Amino-, Thiocyanatgruppe, Halogen oder eine Phosphit- oder Phosphatgruppe oder eine modifizierte Phosphatgruppe, z.B. eine Phosphonat- oder Thiophosphatgruppe, oder eine sonstwie geeignete Funktion darstellt.

**4.** Energietransfersysteme nach einem der Ansprüche 1-3, dadurch gekennzeichnet, dass das Chromophor vom Lumazintyp ein Lumazinderivat der allgemeinen Formel I ist, worin $R_1$ und $R_2$ jeweils für ein H-Atom, eine gegebenenfalls substituierte $C_{1-10}$-Alkylgruppe, 1'-Ribosyl, 1'(2'-Desoxyribosyl) oder den Rest einer analogen Hydroxyverbindung stehen; $R_3$ für ein H-Atom oder für eine gegebenenfalls substituierte $C_{1-10}$-Alkylgruppe steht; und $R_4$ für eine gegebenenfalls substituierte $C_{1-10}$-Alkylgruppe, 1'-Ribosyl, 1'(2'-Desoxyribosyl) oder den Rest einer analogen Hydroxyverbindung steht.

**5.** Energietransfersysteme nach einem der Ansprüche 1-3, dadurch gekennzeichnet, dass das Chromophor vom Lumazintyp ein Lumazinderivat der allgemeinen Formel II ist, worin $R_5$ und $R_6$ für eine gegebenenfalls substituierte $C_{1-10}$-Alkylgruppe stehen; und $R_7$ und $R_8$ für 1'-Ribosyl, 1'(2'-Desoxyribosyl) oder den Rest einer analogen Hydroxyverbindung stehen.

**6.** Energietransfersysteme nach einem der Ansprüche 1-5, dadurch gekennzeichnet, dass ein oder mehrere Chromophore vom Lumazintyp am Ende einer DNS- oder RNS-Sequenz oder innerhalb einer DNS- oder RNS-Sequenz anstelle eines oder mehrerer Nukleoside in der DNS- oder RNS-Sequenz eingebaut sind und die DNS- oder RNS-Sequenz an einen Rutheniumkomplex der allgemeinen Formel III gebunden ist, worin die Ladungsübertragungseinheiten $L_1$, $L_2$ und $L_3$ gleich oder verschieden sind und Bipyridyl, Bathophenanthrolin oder Benzbathophenanthrolin-Gruppen darstellen, die gegebenenfalls substituiert sein können.

**7.** Energietransfersysteme nach Anspruch 6 mit einer der folgenden Formeln

$Ru^{2+}$ $L_1$ $L_2$ $L_3$-d(LuGTTGACAAGAATCCTCACAATACC) 3',
$Ru^{2+}$ $L_1$ $L_2$ $L_3$-d(GTLuGACAAGAATCCTCACAATACC) 3',
$Ru^{2+}$ $L_1$ $L_2$ $L_3$-d(GTTGALuAAGAATCCTCACAATACC) 3',
$Ru^{2+}$ $L_1$ $L_2$ $L_3$-d(GTTGACAALuAATCCTCACAATACC) 3', oder
$Ru^{2+}$ $L_1$ $L_2$ $L_3$-d(LuLuLuLuLuGTTGACAAGAATCCTCACAATACC) 3'

worin der Rutheniumkomplex der allgemeinen Formel III über eine sehr stabile Phosphodiesterbindung an die DNS oder RNS gebunden ist und die Chromophore vom Lumazintyp 1-(2'-desoxy-$\alpha$-D-ribofuranosyl)-6,7-dimethyl-lumazin [Lu] sind.

**8.** Energietransfersysteme nach einem der Ansprüche 1-5, dadurch gekennzeichnet, dass die Chromophore vom Lumazintyp und die Rutheniumkomplexe in verschiedene modifizierte oder nichtmodifizierte DNS- oder RNS-Sequenzen eingebaut sind.

**9.** Verwendung der Energietransfersysteme gemäss den Ansprüchen 1-8 für Abstandsmessungen innerhalb eines Moleküls oder zwischen verschiedenen Molekülen, insbesondere für Abstandsmessungen innerhalb einer DNS- oder RNS-Sequenz oder zwischen verschiedenen DNS- oder RNS-Sequenzen.

**10.** Verwendung nach Anspruch 9 in diagnostischen Tests.

**11.** DNS- oder RNS-Sequenzen, die eine oder mehrere Chromophore vom Lumazintyp enthalten.

**12.** DNS- oder RNS-Sequenzen gemäss Anspruch 11, dadurch gekennzeichnet, dass die Chromophore vom Lumazintyp Lumazinderivate der allgemeinen Formel

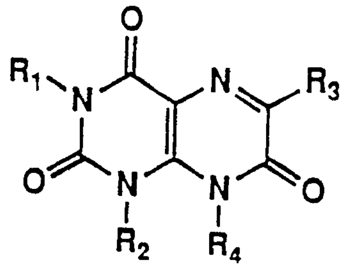

I

worin $R_1$ und $R_2$ jeweils für ein H-Atom, eine gegebenenfalls substituierte $C_{1-10}$-Alkylgruppe, 1'-Ribosyl, 1'(2'-Desoxy-ribosyl) oder den Rest einer analogen Hydroxyverbindung stehen; $R_3$ für ein H-Atom oder für eine gegebenenfalls substituierte $C_{1-10}$-Alkylgruppe steht; und $R_4$ für eine gegebenenfalls substituierte $C_{1-10}$-Alkylgruppe, 1'-Ribosyl, 1'(2'-Desoxyribosyl) oder den Rest einer analogen Hydroxyverbindung steht,
oder der allgemeinen Formel

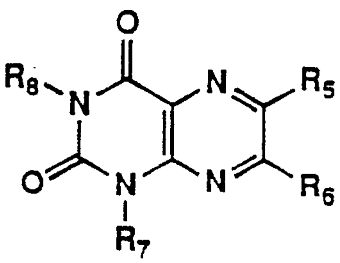

II

worin $R_5$ und $R_6$ jeweils für eine gegebenenfalls substituierte $C_{1-10}$-Alkylgruppe stehen, und $R_7$ und $R_8$ für 1'-Ribosyl, 1'(2'-Desoxyribosyl) oder den Rest einer analogen Hydroxyverbindung stehen, sind.

**13.** DNS-Sequenzen nach Anspruch 12 mit den folgenden Formeln

d(LuGTTGACAAGAATCCTCACAATACC) 3',
d(GTLuGACAAGAATCCTCACAATACC) 3',
d(GTTGALuAAGAATCCTCACAATACC) 3',
d(GTTGACAALuAATCCTCACAATACC) 3',
d(LuLuLuLuLuGTTGACAAGAATCCTCACAATACC) 3',
d(TGGGATAGGTGGATTAT-LuLuLuLu) 3'
d[CTACTGGGATAGGTGGA-LuLuLuLu) 3'
d[TCAACGTATGTTCACCGC-LuLuLuLu) 3,.

**14.** DNS-Sequenzen nach Anspruch 13, dadurch gekennzeichnet, dass die Chromophore vom Lumazintyp (Lu) 1-(2'-desoxy-α-D-ribofuranosyl)-6,7-dimethyl-lumazin sind.

**15.** Lumazinderivate der allgemeinen Formel

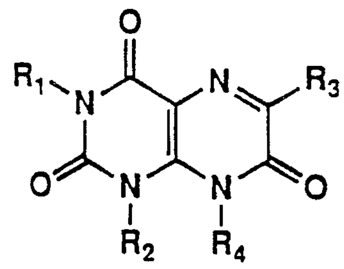

I

worin $R_1$ und $R_2$ jeweils für ein H-Atom, eine gegebenenfalls substituierte $C_{1-10}$-Alkylgruppe, 1'-Ribosyl, 1'(2'-Desoxyribosyl) oder den Rest einer analogen Hydroxyverbindung stehen; $R_3$ für ein H-Atom oder für eine gegebenenfalls substituierte $C_{1-10}$-Alkylgruppe steht; und $R_4$ für eine gegebenenfalls substituierte $C_{1-10}$-Alkylgruppe, 1'-Ribosyl, 1'(2'-Desoxyribosyl) oder den Rest einer analogen Hydroxyverbindung steht,
oder der allgemeinen Formel

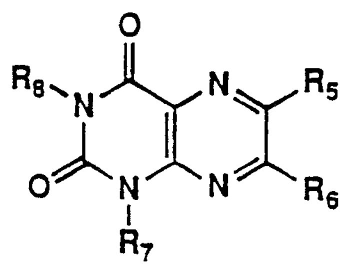

II

worin $R_5$ und $R_6$ jeweils für eine gegebenenfalls substituierte $C_{1-10}$-Alkylgruppe stehen, und $R_7$ und $R_8$ für 1'-Ribosyl, 1'(2'-Desoxyribosyl) oder den Rest einer analogen Hydroxyverbindung stehen.

**16.** Phosphoramidite oder H-Phosphonate der Lumazinderivate nach Anspruch 15, insbesondere 1-(5'-0-4,4'-Dimethoxy-trityl-2'-desoxy-α-D-ribofuranosyl-3 '-0-((2-cyanoethyl)-N,N-diisopropyl-phosphoramidit)-6,7-dimethyl-lumazin.

**17.** Mit Lumazinderivaten gemäss Anspruch 15 modifiziertes Trägermaterial, insbesondere 1-(5'-0-4,4'-Dimethoxy-trityl-2'-desoxy-α-D-ribofuranosyl-3'-0-succinyl)-6,7-di-methyl-lumazin.

**18.** Verwendung des Trägermaterials nach Anspruch 17 in der Festphasensynthese.

**19.** Verfahren zur Herstellung von Energietransfersystemen, bestehend aus zwei organischen Verbindungen, von denen die eine ein Chromophor vom Lumazintyp ist und die andere, mit der sie in Wechselwirkung stehn, ein Ru-Komplex ist, dadurch gekennzeichnet, die beiden Chromophore nach beschriebenen Methoden unabhängig voneinander synthetisiert werden und für die Wechselwirkung in räumliche Nähe gebracht werden.

**20.** Verfahren zur Herstellung von DNS- oder RNS-Sequenzen, die eine oder mehrere Chromophore vom Lumazintyp enthalten, dadurch gekennzeichnet, dass Lumazinderivate der allgemeinen Formel

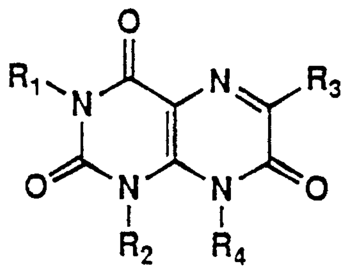

I

worin $R_1$ und $R_2$ jeweils für ein H-Atom, eine gegebenenfalls substituierte $C_{1-10}$-Alkylgruppe, 1'-Ribosyl, 1'(2'-Desoxyribosyl) oder den Rest einer analogen Hydroxyverbindung stehen; $R_3$ für ein H-Atom oder für eine gegebenenfalls substituierte $C_{1-10}$-Alkylgruppe steht; und $R_4$ für eine gegebenenfalls substituierte $C_{1-10}$-Alkylgruppe, 1'-Ribosyl, 1'(2'-Desoxyribosyl) oder den Rest einer analogen Hydroxyverbindung steht,
oder der allgemeinen Formel

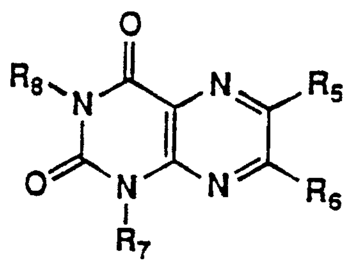

II

worin $R_5$ und $R_6$ jeweils für eine gegebenenfalls substituierte $C_{1-10}$-Alkylgruppe stehen, und $R_7$ und $R_8$ für 1'- Ribosyl, 1'(2'-Desoxyribosyl) oder den Rest einer analogen Hydroxyverbindung stehen, in einer für die Kopplung geeigneten Form, vorzugsweise als Phosphoramidite, H-Phosphonate oder aktivierte Phosphatfunktionen im Verlauf der DNS- oder RNS-Synthese in die zu synthetisierenden Sequenzen kovalent eingebaut werden.

**21.** Verfahren zur Herstellung von mit Lumazinderivaten verknüpftem Trägermaterial, dadurch gekennzeichnet, dass Lumazinderivate der allgemeinen Formel

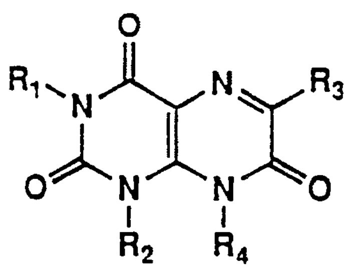

I

worin $R_1$ und $R_2$ jeweils für ein H-Atom, eine gegebenenfalls substituierte $C_{1-10}$-Alkylgruppe, 1'-Ribosyl, 1'(2'-Desoxyribosyl) oder den Rest einer analogen Hydroxyverbindung stehen; $R_3$ für ein H-Atom oder für eine gegebenenfalls substituierte $C_{1-10}$-Alkylgruppe steht; und $R_4$ für eine gegebenenfalls substituierte $C_{1-10}$-Alkylgruppe, 1'-Ribosyl, 1'(2'-Desoxyribosyl) oder den Rest einer analogen Hydroxyverbindung steht,
oder der allgemeinen Formel

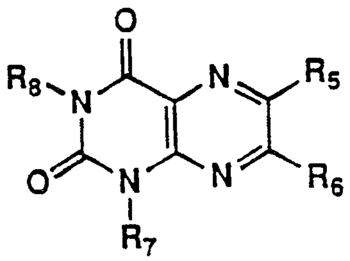

II

worin $R_5$ und $R_6$ jeweils für eine gegebenenfalls substituierte $C_{1-10}$-Alkylgruppe stehen, und $R_7$ und $R_8$ für 1'-Ribosyl, 1'(2'-Desoxyribosyl) oder den Rest einer analogen Hydroxyverbindung stehen, nach Überführung in eine für die Verknüpfung mit dem Träger geeignete Form, vorzugsweise als Succinylderivat, mit diesem unter Bildung einer kovalenten Bindung gekoppelt werden.

**22.** Energietransfersysteme nach Anspruch 1, hergestellt nach einem Verfahren gemäss Anspruch 19.

**23.** DNS- und RNS-Sequenzen nach Anspruch 12, hergestellt nach einem Verfahren gemäss Anspruch 20.

**24.** Trägermaterial nach Anspruch 17, hergestellt nach einem Verfahren gemäss Anspruch 21.

# FIG 1

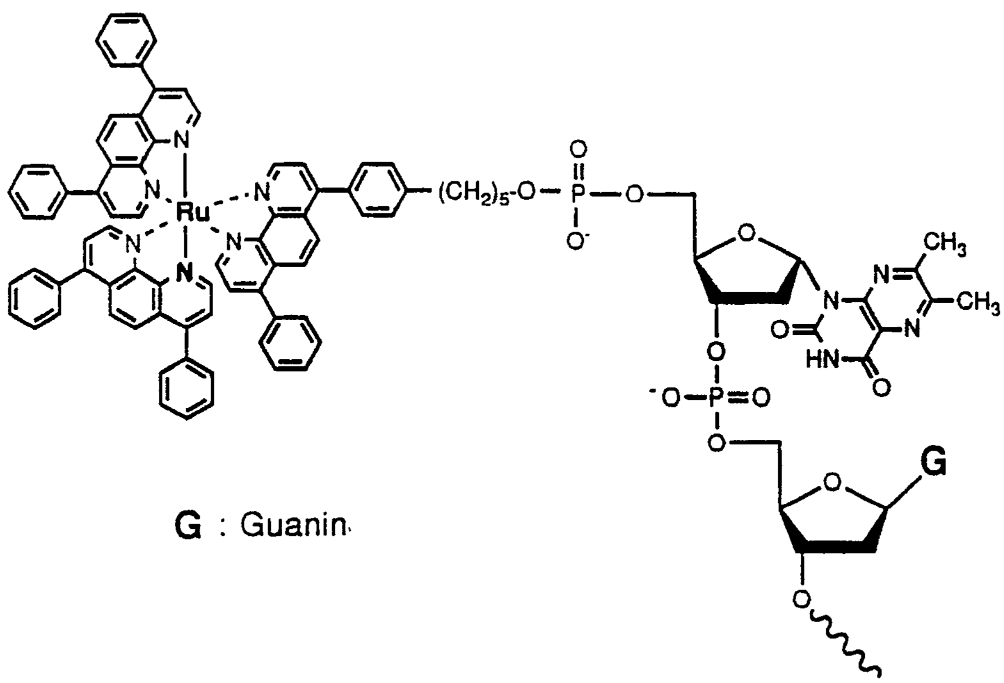

G : Guanin

# FIG 2

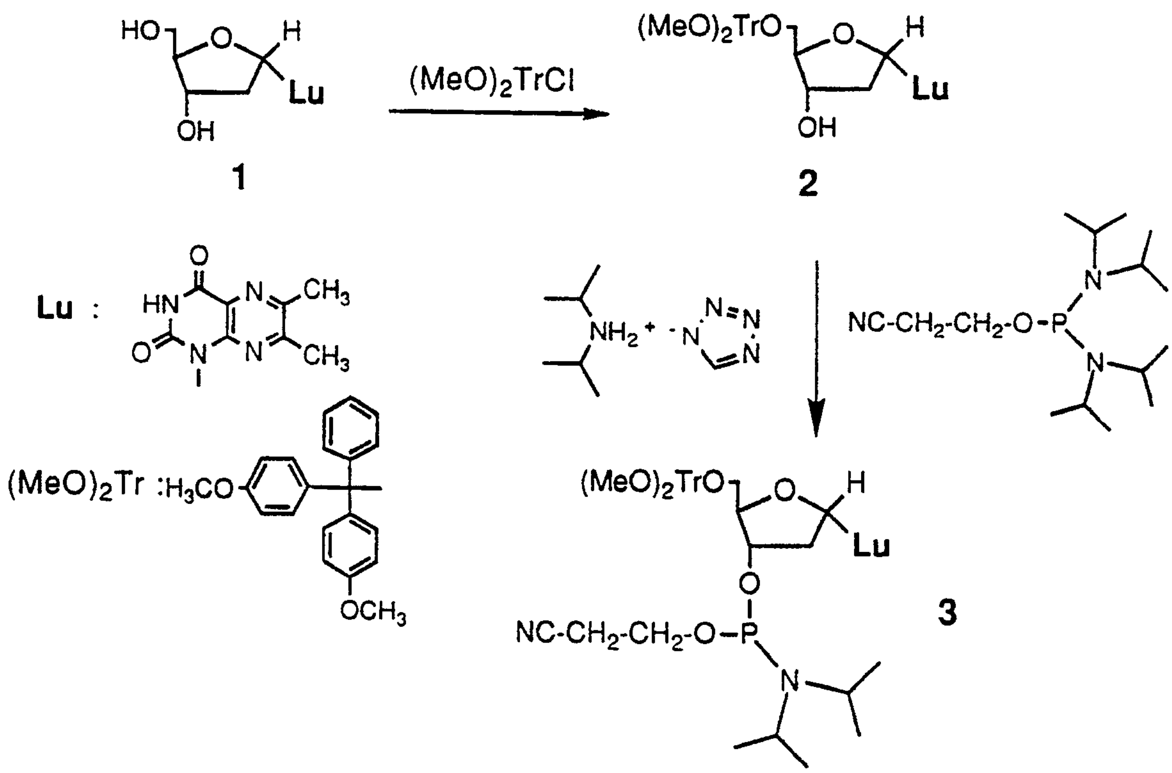


1

2

3

Lu :

(MeO)2Tr :

# FIG 3

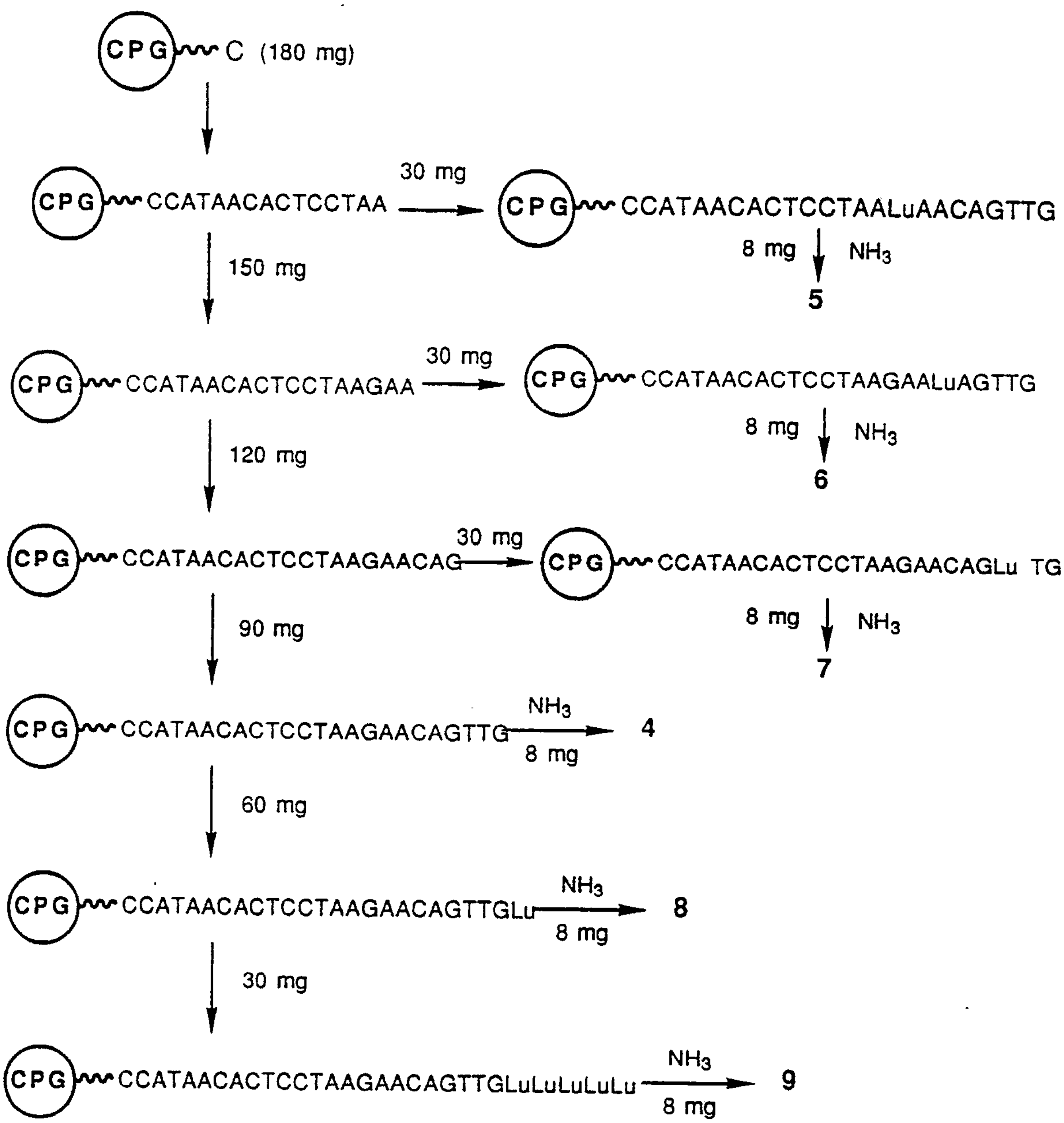
CPG C (180 mg)
CPG CCATAACACTCCTAA
30 mg
CPG CCATAACACTCCTAALuAACAGTTG
8 mg
NH3
5
150 mg
CPG CCATAACACTCCTAAGAA
30 mg
CPG CCATAACACTCCTAAGAALuAGTTG
8 mg
NH3
6
120 mg
CPG CCATAACACTCCTAAGAACAG
30 mg
CPG CCATAACACTCCTAAGAACAGLu TG
8 mg
NH3
7
90 mg
CPG CCATAACACTCCTAAGAACAGTTG
NH3
8 mg
4
60 mg
CPG CCATAACACTCCTAAGAACAGTTGLu
NH3
8 mg
8
30 mg
CPG CCATAACACTCCTAAGAACAGTTGLuLuLuLuLu
NH3
8 mg
9

FIG 4

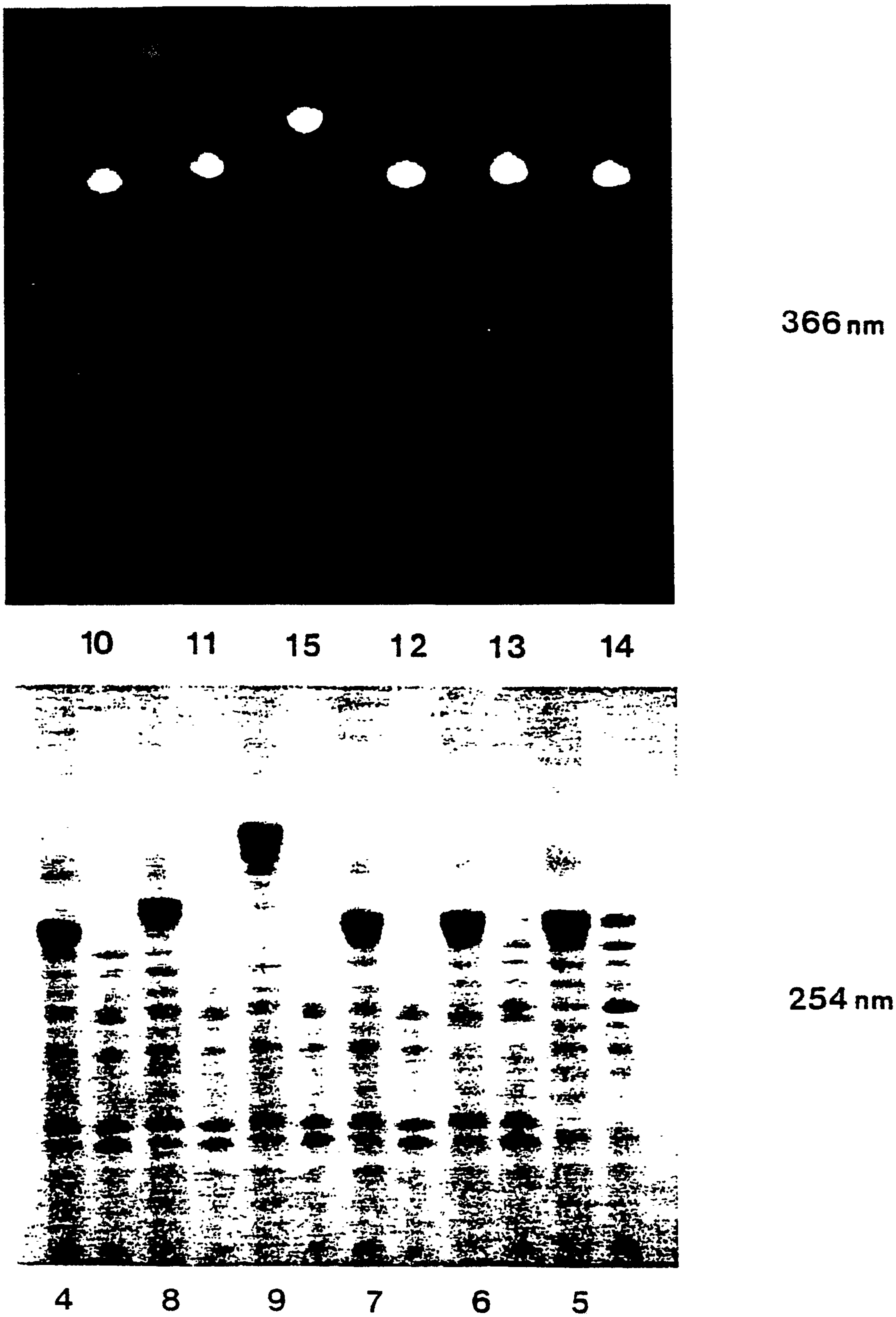

# FIG 5

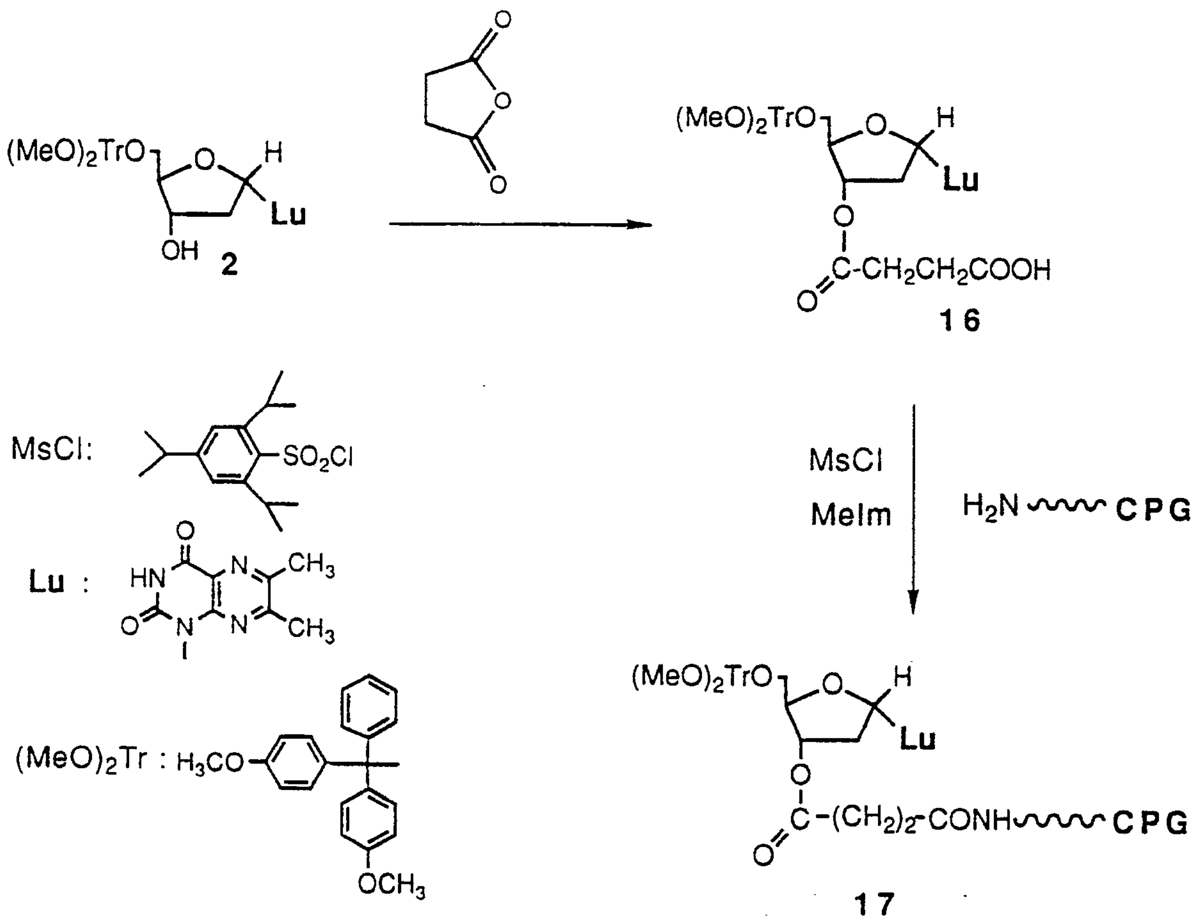


2

16

17

MsCl:

Lu :

(MeO)2Tr :

# FIG 6

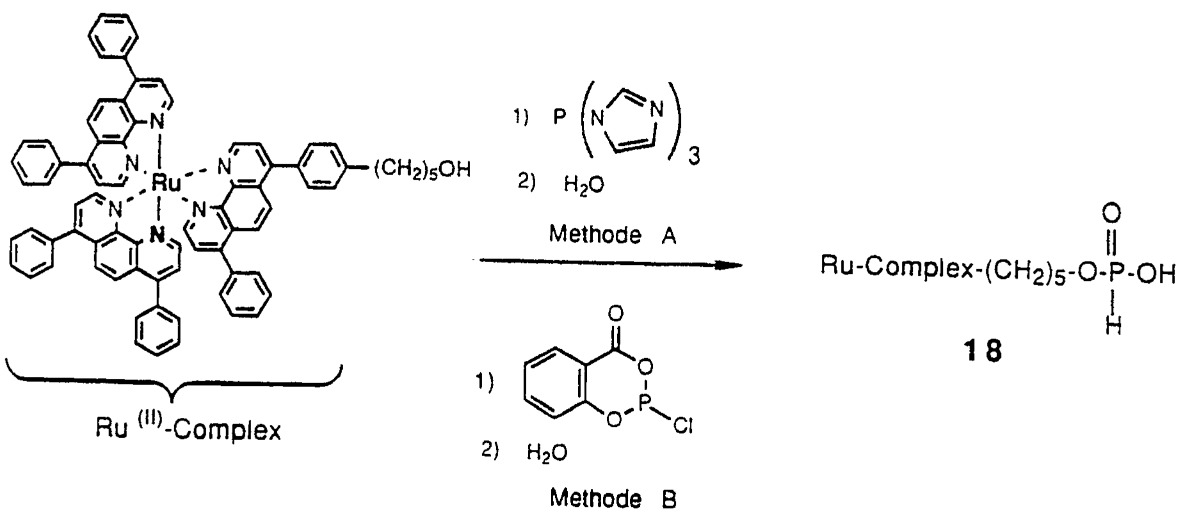
1) P
3
2) $H_2O$
Methode A
1)
2) $H_2O$
Methode B
Ru-Complex-$(CH_2)_5$-O-P-OH
Ru (II)-Complex

18

# FIG 7

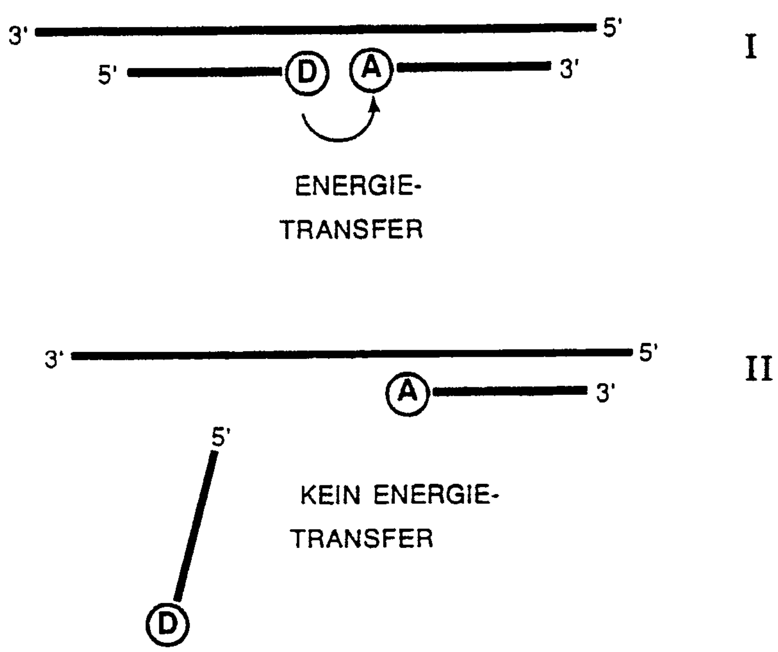


d(ACCCTATCCACCTAATAAAAATATTAGGTGGATAGGGTCATCCTCTTTA)5'
**22**

d(TGGGATAGGTGGATTAT-$Lu_4$) Ru-d(ATAATCCACCTATCCCAGTAGGAGAAAT)
**20** **21**

ENERGIE-TRANSFER

d(CTACTGGGATAGGTGGA-$Lu_4$) **23**

d(TCAACGTATGTTCACCG-$Lu_4$) **24**

d(TGGGATAGGTGGATTAT) **25**

(24 and 25: Kontrollen)

# FIG 8

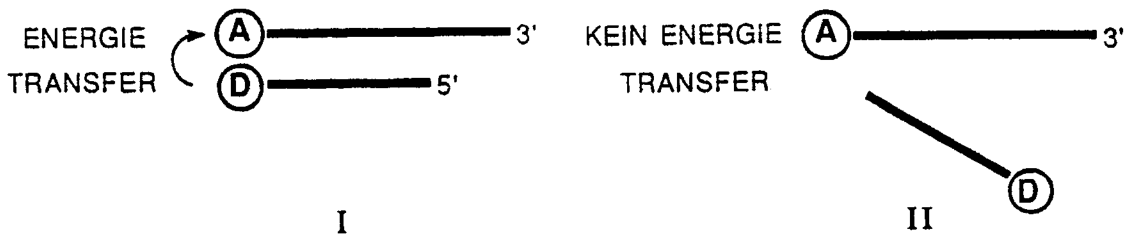


Ru-d(ATAATCCACCTATCCCAGTAGGAGAAAT)$_{3'}$ **21**

d(Lu$_4$-TATTAGGTGGATAGGGT)$^{5'}$ **20**

d(Lu$_4$-AGGTGGATAGGGTCATC)$^{5'}$ **23**

d(Lu$_4$-GCCACTTGTATGCAACT)$^{5'}$ **24**

d(TATTAGGTGGATAGGGT)$^{5'}$ **25**

} Kontrollen